Europäisches Patentamt

European Patent Office

Office européen des brevets

(11) Publication number: **0 476 199 A1**

## EUROPEAN PATENT APPLICATION

(21) Application number: **90310369.5**

(22) Date of filing: **21.09.90**

(51) Int. Cl.5: **C07K 15/00**, C07K 1/14, C07K 3/22, A23J 3/32, A23J 3/34

(43) Date of publication of application:
**25.03.92 Bulletin 92/13**

(84) Designated Contracting States:
**AT BE CH DE DK ES FR GB GR IT LI LU NL SE**

(71) Applicant: **UNILEVER PLC**
**Unilever House Blackfriars**
**London EC4P 4BO(GB)**

(72) Inventor: **Kunst, Anthonie**
**Weegbree 41**
**1273 AN Huizen(NL)**

(74) Representative: **Hartong, Richard Leroy et al**
**Unilever N.V. Patent Division P.O. Box 137**
**NL-3130 AC Vlaardingen(NL)**

(54) Process to isolate phosphopeptides.

(57) A method for the separation of phosphopeptides from casein hydrolysates is described comprising the steps of:

i) adjusting the pH of an aqueous medium containing sodium caseinate to 4.6 and separation of the solids therefrom;

ii) adjusting the pH of the aqueous medium derived from step (i) to a value > 6.0;

iii) subjecting the aqueous medium derived from step (ii) to ion exchange thereby to saturate the resin and collecting the unbound material;

iv) eluting the ion exchange resin with a suitable eluent; and

v) drying the product obtained from step iv).

This invention relates to the isolation of phosphopeptides from enzymic hydrolysates of casein. Patent application WO/87/07615 (The University of Melbourne et al) provides a phosphopeptide or a salt thereof, the phosphopeptide having 5 to 30 amino acids including the sequence A-B-C-D-E where A, B, C, D, E are independently phosphoserine, phosphothreonine, phosphotyrosine, phosphohistidine, glutamate and aspartate. These phosphopeptides may have utility in the treatment or inhibition of dental diseases. Patent application WO/87/07615 describes two extraction/fractionation methods to obtain the phosphopeptides. One method is based on the use of ion-exchange, the other method is based on a selective precipitation using ethanol and acetone.

Both methods suffer from the disadvantage that application on an industrial scale is not possible at acceptable costs.

The preferred isolation method is ion exchange chromatography. The method described in WO/87/07615 allows the isolation of only very small quantities on a laboratory scale and makes use of a ion exchange resin (MONO Q ex Pharmacia/LKB) which is not generally recommended by its manufacturer for use on an industrial scale. In addition the method involves the use of TRIS (2-amino-2-hydroxymethyl-1,3-propane diol) as a buffer substance to control the pH which is generally regarded as essential if reproducible results are to be obtained. TRIS is however not allowed in food, feed, cosmetic and pharmaceutical products and have to be removed after the ion exchange process which is not attractive from an economical point of view.

To produce phosphopeptides economically on an industrial scale we therefore had to develop an alternative method. Since the phosphopeptides have a unique charge density (the isoelectric point is about 4) anion exchange chromatography is the preferred method.

The operating conditions for the anion exchange column and the particle size of the resin should be such in relation to the desired throughput as to maintain a pressure drop accross the column of less than 3 bar.

The phosphopeptides are bound to the anion exchange resin at a pH >4.0 and eluted either at a pH < 4.0 or at a pH > 4.0 in the presence of a suitable salt in a sufficient high concentration.

To avoid blockage of the columns it is strongly recommended to perform a pretreatment before the ion exchange step to remove insoluble components from the casein hydrolysate. A suitable method is adjusting the pH of the medium containing the casein hydrolysate to 4.0 - 6.0, preferably 4.6 and separation of the solids therefrom.

The ion exchange process is carried out after adjusting the pH of the clear medium containing the peptides to a value > 4.0. The column is loaded with the phosphopeptide containing medium until it is saturated, while collecting the non-bound material. The non-bound material can subsequently be dried.

The ion exchange resin is eluted with a suitable eluent to collect the bound material.

Suitable eluents are an aqueous medium with a pH < 4.0 or an aqueous medium with a pH > 4.0 and a salt concentration higher than 100 mMol, preferably 500 mMol.

After collecting the eluted material which contains the phosphopeptides the product is dried.

In case salt is used in the eluent to collect the phosphopeptide material this is removed before drying.

EXAMPLE 1

A chromatography column with an internal diameter of 2.6 cm is packed with a slurry of Matrex Silica PAE 300 weak anion exchanger until a bed height of 10 cm is reached.

A 10% solution of sodium caseinate is hydrolysed with pancreatin. The insoluble fraction is removed via adjustment of the pH to 4.6 and a subsequent centrifugation at 5000 G.

The pH of the obtained solution is adjusted to 8.0. The peptides in the solution are then fractionated on the (an)ion exchange column.

The unbound peptides are collected and are dried.

The peptides which bind to the ion exchange resin are eluted with a 1M sodium chloride solution with a pH of 8.0 and are collected. The obtained aqueous medium which contains the phosphopeptides, is desalinated and is subsequently dried.

EXAMPLE 2

A chromatography column with an internal diameter of 2.6 cm is packed with a slurry of Matrex Silica PAE 300 weak anion exchanger until a bed height of 10 cm is reached.

A 10% solution of sodium caseinate is hydrolysed with pancreatin. The insoluble fraction is removed via adjustment of the pH to 4.6 and a subsequent centrifugation at 5000 G.

The pH of the obtained solution is adjusted to 8.0. The peptides in the solution are then fractionated on the (an)ion exchange column.

The unbound peptides are collected and are dried.

The peptides which bind to the ion exchange resin are eluted with an aqueous medium with a pH of 1.0 and are collected. The pH of the obtained aqueous medium which contains the phosphopeptides, is adjusted to 7.0 and is subsequently dried.

## Claims

1.  A method for the separation of phosphopeptides from casein hydrolysates comprising the steps of:

    i) adjusting the pH of an aqueous medium containing sodium caseinate to 4.0 - 6.0, preferably 4.6 and separation of the solids therefrom;

    ii) adjusting the pH of the aqueous medium derived from step (i) to a value > 4.0, preferably > 6.0;

    iii) subjecting the aqueous medium derived from step (ii) to ion exchange thereby to saturate the resin and collecting the unbound material;

    iv) eluting the ion exchange resin with a suitable eluent;

    v) drying the product obtained from step iv).

2.  A method according to Claim 1, wherein the eluent used in step iv) is an aqueous medium with a pH < 4.0 or an aqueous medium with a pH > 4.0 and a salt concentration higher than 100 mMol, preferably 500 mMol.

3.  A method according to claim 1 wherein the ion exchange resin is eluted in step (iv) with a sodium chloride solution and the aqueous medium so obtained is desalinated before drying.

4.  A method according to claim 1 wherein the ion exchange resin is anionic and is eluted in step (iv) with aqueous hydrochloric acid, preferably having a pH of from 1 to 3.

5.  A method according to claim 1 to 3 wherein the step (i) is omitted.

6.  A method according to claim 1, carried out in the absence of TRIS.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | CHEMICAL ABSTRACTS, vol. 111, no. 13, 25th September 1989, page 551, abstract no. 113883u, Columbus, Ohio, US; M.A. JUILLERAT et al.: "Tryptic phosphopeptides from whole casein. I. Preparation and analysis by fast protein liquid chromatography", & J. DAIRY RES. 1989, 56(4), 603-11 * Abstract * | 6 | C 07 K 15/00 C 07 K 1/14 C 07 K 3/22 A 23 J 3/32 A 23 J 3/34 |
| | – – – | | |
| Y | IDEM | 1-5 | |
| | – – – | | |
| X | CHEMICAL ABSTRACTS, vol. 108, no. 23, 6th June 1988, page 572, abstract no. 203481d, Columbus, Ohio, US; F. TOURAINE et al.: "Chromatographic separation of peptides from enzymic hydrolysis of whey proteins and caseins", & LAIT 1987, 67(4), 419-36 * Abstract * | 6 | |
| | – – – | | |
| Y | IDEM | 1-5 | |
| | – – – | | |
| Y | JOURNAL OF THE AMERICAN OIL CHEMISTS' SOCIETY (JAOCS), vol. 64, no. 12, December 1987, pages 1704-1711, Champaign, IL, US; J.-M. CHOBERT et al.: "Specific limited hydrolysis and phosphorylation of food proteins for improve-ment of functional and nutritional properties" * Whole document, especially page 1706, right-hand column, lines 15-30; figure 2 * | 1-5 | **TECHNICAL FIELDS SEARCHED (Int. Cl.5)** C 07 K A 23 J |
| | – – – | | |
| A | BIOLOGICAL ABSTRACTS, vol. 64, no. 11, 1977, abstract no. 62292, Philadelphia, PA, US; D.W. WEST: "A simple method for the isolation of a phosphopeptide from bovine alphas1-casein", & DAIRY RES. 44(2): 373-376. 1977 * Abstract * | 1,6 | |
| | – – – – – | | |

The present search report has been drawn up for all claims

| Place of search | Date of completion of search | Examiner |
|---|---|---|
| The Hague | 21 May 91 | MASTURZO P. |